# EUROPÄISCHE PATENTSCHRIFT

(11) **EP 1 265 589 B1**
(45) Veröffentlichungstag und Bekanntmachung des Hinweises auf die Patenterteilung: **11.10.2006**
(21) Anmeldenummer: 01933701.3
(22) Anmeldetag: 15.03.2001
(51) Int. Cl.: A61K 8/22, A61K 8/25, A61K 8/44, A61Q 11/00

(54) **SUPRAGINGIVALES PULVERSTRAHLEN**
SUPRAGINGIVAL POWDER SPRAYING
PROJECTION DE POUDRE DANS LA ZONE SUPRAGINGIVALE

(30) Priorität: 24.03.2000 DE 10014416
(43) Veröffentlichungstag der Anmeldung: 18.12.2002
(73) Patentinhaber: 3M ESPE AG, 82229 Seefeld (DE)
(72) Erfinder: FLEMMIG, Thomas, 48161 Münster (DE); GANGNUS, Bernd, 82346 Andechs (DE); GASSER, Oswald, 82229 Seefeld (DE); GUGGENBERGER, Rainer, 82211 Herrsching (DE); HÄBERLEIN, Ingo, 82362 Weilheim (DE); WINDMÜLLER, Bettina, 82205 Gilching (DE)
(86) Internationale Anmeldenummer: PCT/EP2001/002893
(87) Internationale Veröffentlichungsnummer: WO 2001/072273

(56) Entgegenhaltungen:
- WO-A-00/53154
- DE-A- 2 930 836
- GB-A- 988 513
- GB-A- 1 480 594
- PATENT ABSTRACTS OF JAPAN vol. 016, no. 081 (C-0915), 27. Februar 1992 (1992-02-27) & JP 03 271215 A (KAO CORP), 3. Dezember 1991 (1991-12-03)

## Beschreibung

Die vorliegende Erfindung betrifft die Verwendung von feinkörnigen Pulvern in einem Verfahren zur Reinigung von Zahnoberflächen.

Im Rahmen einer modernen Prophylaxebehandiung ist es heute üblich, die Zahnhartsubstanz von harten und weichen Belägen zu reinigen. Dabei werden zur Entfernung der harten Beläge, auch Kongremente oder Zahnstein genannt, üblicherweise mechanische Verfahren, wie Abkratzen mit entsprechenden Handinstrumenten, oder der Einsatz von Ultraschallspitzen zur Anwendung gebracht.

Die sog. weichen Beläge, z.B. verursacht durch Genuss von Kaffee oder Zigaretten, werden meist entweder mit Polierpasten oder durch das Abstrahlen mit einem Pulver-Wasser-Gemisch entfernt. Das Abstrahlen wird dabei üblicherweise mit einem Gemisch aus Natriumbicarbonatpulver in Wasser durchgeführt. Dies bietet gegenüber dem Polieren mit Polierpasten den Vorteil, dass sich die Beläge deutlich schneller entfernen lassen. Nachteilig ist jedoch, dass die eingesetzten Pulver-Wasser-Gemische gegenüber der Zahnoberfläche eine abrasive Wirkung zeigen, wodurch die behandelten Zahnflächen aufgerauht werden. Dadurch wird ein weiterer Arbeitsschritt notwendig, in dem mittels Polierscheiben die Zahnoberfläche wieder geglättet werden muss. Darüber hinaus führt diese Abrasivität der eingesetzten Pulver-Wasser-Gemische zu einem Abtrag an Zahnhartsubstanz, der bei wiederholter Anwendung zu Sensibilitäten, insbesondere im Zahnhalsbereich führen kann.

Die GB-A-1 480 594 offenbart ein Verfahren zur Reinigung von Zahnoberflächen mit einem abrasive Substanzen enthaltenden Wasserstrahl. Als geeignete Substanzen werden beschichtete oder unbeschichtete, organische oder anorganische Substanzen genannt. Ein Hinweis dahingehend, welche Substanzen tatsächlich eine effektive und schonende Reinigung der Zahnoberfläche ermöglichen, fehlt.

Im Gegensatz dazu wird in der deutschen Patentanmeldung 199 10 559 die Verwendung feinkörniger Pulver zur Herstellung eines Mittels für die Pulverstrahlreinigung von Zahnwurzeloberflächen, d.h. subgingivaler Zahnoberflächen beschrieben.

Aufgabe der vorliegenden Erfindung ist es, Pulver bzw. Pulvergemische für die Reinigung von Zahnoberflächen zur Verfügung zu stellen, die die genannten Probleme vermeiden.

Gelöst wird die Aufgabe durch die Verwendung von Pulvern bzw. Pulvergemischen, wie sie in den Ansprüchen beschrieben werden.

Die erfindungsgemäß verwendeten Pulver bzw. Pulvergemische sind so beschaffen, dass die durch Pulverbestrahlung verursachte Abrasion der supragingivalen Zahnoberfläche nicht mehr als 0,10 mm³, vorzugsweise nicht mehr als 0,08 mm³, besonders bevorzugt nicht mehr als 0,05 mm³ beträgt, bezogen auf eine Zahnoberfläche von 9,6 mm² bei einer Bestrahlungsdauer von 2 min, bei einem Strahldruck von 4 bar und einem Abstand zwischen Zahnoberfläche und Strahldüse von nicht mehr als 2,5 mm.

Erfindungsgemäß verwendbare Pulver bzw. Pulvergemische weisen eine Dichte von nicht mehr als 2,0 g/cm³ auf und haben eine mittlere Korngröße von nicht mehr als 45 µm.

Pulver mit diesen Eigenschaften zeigen trotz einer guten Reinigungswirkung keine nennenswerte Abrasivität gegenüber der supragingivalen Zahnhartsubstanz bzw. der sichtbaren Zahnoberfläche, die sonst üblicherweise bei bekannten Mitteln auftritt.

Die Abrasivität ist dabei so gering, dass ein Volumenabtrag an der supragingivalen Zahnhartsubstanz, insbesondere dem Zahnschmelz oder Enamel, mit auf dem Dentalsektor üblichen Dubliermassen (beispielsweise Dimension® Garant, Fa. ESPE) und anschließender lichtmikroskopischer Untersuchung des Abdrucks nicht, bzw. in nur sehr geringem Umfang festgestellt werden kann.

Dadurch erübrigt sich ein nachgeschalteter Polierschritt.

Ferner können diese Pulver bzw. Pulvergemische wiederholt, auch in kurzen Abständen, angewandt werden, ohne dass es zu einem nennenswerten Verlust an nicht regenerierbarer Zahnhartsubstanz kommt. Die zeitlichen Abstände können dabei auch nur wenige Tage oder Wochen betragen.

Überraschenderweise wurde gefunden, dass sich mit den Pulvern bzw. Pulvergemischen nicht nur übliche Verunreinigungen und Verfärbungen der sichtbaren Zahnhartzubstanz, die beispielsweise von Nikotin-, Kaffee-, Tee- oder Rotweinfarbstoffen herrühren, sondern auch nicht oder nur schlecht sichtbare Plaque-Reste, insbesondere Ablagerungen, die von Mikroorganismen herrühren, entfernen lassen.

Bedingt durch die vorzugsweise kleine mittlere Korngröße gelingt die Reinigung der Zahnoberflächen auch in Interdentalräumen.

Eine hohe Effizienz der Pulverstrahlreinigung wird beispielsweise dann erreicht, wenn vor dem eigentlichen Reinigungsschritt die Zahnsubstanz auf insbesondere nichtsichtbare Plaque-Reste hin untersucht wird. Solche Ablagerungen auf der Zahnsubstanz werden durch herkömmliches Zähneputzen üblicherweise nicht entfernt.

Eine Detektion solcher Ablagerungen gelingt beispielsweise durch Verwendung von geeigneten Abdruckmassen, wie sie in der deutschen Patentanmeldung 199 26 728 beschrieben sind.

Die dort beschriebenen verformbaren, härtbaren und/oder filmbildenden Trägermaterialien enthalten für die orts- und stoffspezifische intraorale Diagnose diagnostisch nutzbare Zusatzstoffe, beispielsweise in einer Konzentration von 0,0001 bis 10 Gew.-%, vorzugsweise in einer Konzentration von 0,01 bis 1 Gew.-%. Als diagnostische Zusätze werden beispielsweise Farbstoffindikatoren, Antikörper und Enzyme genannt. Diese können gegebenenfalls in mikroverkapselter Form vorliegen.

Geeignet sind beispielsweise Abdruckmassen auf Polyetherbasis, Silikonbasis, Hydrokolloidbasis oder Alginatbasis.

Im folgenden wird die Erfindung näher erläutert.

Pulver bzw. Pulvergemische, die sich für die Verwendung in der vorliegenden Erfindung eignen, sind insbesondere solche, die sich mittels herkömmlicher Pulverstrahlgeräte für den Dentalbereich fördern lassen.

Allen für die erfindungsgemäße Verwendung geeigneten Pulvern ist gemeinsam, dass sie üblicherweise eine geringere Dichte aufweisen als bislang eingesetzte Pulver bzw. Pulvergemische, die für die supragingivale Zahnreinigung verwendet werden.

Femer weisen sie vorzugsweise eine kleine mittlere Korngröße von nicht mehr als 45 µm auf.

Bevorzugt sind auch Pulver mit einer Kornverteilung von 0,05 µm bis 60 µm, besonders bevorzugt mit einer Kornverteilung von 0,1 µm bis 40 µm.

Selbstverständlich sind auch Pulvergemische aus mindestens zwei Pulvern für den beschriebenen Zweck geeignet. Das Mischungsverhältnis ist dabei grundsätzlich beliebig, liegt aber bei Verwendung zweier Pulver vorzugsweise im Bereich von 1 : 10 bis 10 : 1 bezogen auf die Masse der zu mischenden Pulver.

Dem Pulver bzw. Pulvergemisch ist ein weiterer, sehr feinteilig vorliegender, Stoff zugemischt, bevor es als Reinigungsmittel für Zahnoberflächen verwendet wird. Dies bewirkt, dass sich die dabei entstehenden Pulvermischungen mit herkömmlichen Pulverstrahlgeräten besser und schneller fördern lassen.

Diese Substanzen werden üblicherweise in einer Menge von 0,01 bis 5,0 Gew.-%, vorzugsweise in einer Menge von 0,5 bis 1 Gew.-% zugesetzt.

Beispiele für solche sehr feinteilig vorliegende Pulver umfassen Boroxid, Kieselgel, hochdisperse Kieselsäure, vorzugsweise silanisiert, beispielsweise mit Organosilanen, wie Kieselsäuren mit Trimethylsilylgruppen.

Die feinkörnigen Pulver haben vorzugsweise eine durchschnittliche Korngröße von ca. 0,07 µm, besonders bevorzugt von ca. 0,02 µm.

Denkbar ist auch die Zumischung von anderen feinteiligen Substanzen, beispielsweise von Bleichmitteln, wie Perborate (z.B. Natriumperborat), fluoridfreisetzenden Substanzen, wie Natriumfluorid, Analgetika, wie Articain oder Lidocain, Bakterioziden, wie Chlorhexidin oder Triclosan, Geschmacksstoffen, wie Zitronensäure oder Ascorbinsäure.

Vorzugsweise werden Pulver bzw. Pulvergemische verwendet, die überwiegend toxikologisch unbedenklich und/oder sowohl im Körper als auch ausserhalb biologisch leicht abbaubar sind.

Geeignete Pulver für die Reinigung von supragingivalen Zahnoberflächen sind insbesondere organische, natürlich vorkommende Substanzen, wie Aminosäuren, Zucker, organische Säuren und deren Salze, wie Alkali- (z.B. Lithium, Natrium, Kalium), Eralkali- (z.B. Magnesium, Strontium) oder Ammoniumsalze. Geeignet sind aber auch anorganische Substanzen, wenn sie die gewünschte geringe Abrasivität gegenüber der supragingivalen Zahnhartsubstanz und vorzugsweise die beschriebene Dichte und Korngröße aufweisen.

Besonders vorteilhaft sind Glycin, Harnstoff, Kaliumhydrogenphthalat und/oder Kalium-Glukonat.

Die Pulver können gegebenenfalls auch oberflächenbeschichtet sein. Als geeignete Oberflächenbeschichtungsmittel seien genannt: Stärke, Alginate, Collagen (Gelatine), Hydrogele, Polyanhydride, Polyester, Polyiminocarbonate, Polycaprolactone, Polyaminosäuren, Polyphosphazene.

Geeignete Pulvermischungen sind beispielsweise Mischungen aus Aminosäuren und Zuckern und/oder organischen Säuren, vorzugsweise eine Mischung aus Glycin mit Harnstoff.

Geeignete handelsübliche, nicht-toxische Pulver der gewünschten Dichte und mit hoher Reinheit werden üblicherweise zunächst in einer Kugelmühle oder Achatscheibenmühle auf die gewünschte Korngröße gemahlen und gesiebt.

Die angegebenen Dichtewerte korrelieren mit den von den Herstellern angegebenen Dichtewerten bzw. sind gängigen Nachschlagewerken entnommen. Die Korngrößen wurden über ein Granulometer ermittelt.

Anschließend werden gegebenenfalls weitere feinteilige Substanzen zugemischt, gegebenenfalls erneut gemahlen und erneut gesiebt.

Das erhaltene Pulvergemisch wird in ein handelsübliches Pulverstrahlgerät eingebracht und üblicherweise mit Hilfe eines Wasserstrahls auf die supragingivale Zahnoberfläche aufgestrahlt.

Denkbar ist aber auch die Verwendung der beschriebenen Pulver oder Pulvergemische zum Reinigen bzw. Abstrahlen von Dentalmaterialien, wie Kronen, Verblendungen und/oder Brücken, die sich außerhalb der Mundhöhle befinden.

Die erfindungsgemäße Verwendung der beschriebenen Pulver bzw. Pulvergemische erfolgt vorzugsweise derart, dass zunächst über ein Diagnosesystem, beispielsweise eine sogenannte diagnostische Abformmasse, wie sie in der deutschen Patentanmeldung 199 26 728 beschrieben ist, ein Negativabdruck des Gebisses angefertigt wird.

Anhand dieses Abdruckes lassen sich die Stellen der Zahnsubstanz bestimmen, an denen sich unerwünschte Ablagerungen, insbesondere mit dem menschlichen Auge nichtsichtbare Ablagerungen, beispielsweise Plaque-Reste und/oder für die Zahnsubstanz schädliche mikrobielle Abbauprodukte befinden.

Denkbar ist aber auch die Anwendung von anderen Diagnosesystemen, die auf Einfärbung der unerwünschten Ablagerungen, beispielsweise über Fluoreszenzfarbstoffe und deren Detektion beruhen. Solche Diagnosesysteme sind beispielsweise in der DE-A-42 00 741 und der DE-A-29 13 415 beschrieben.

Im Anschluss daran erfolgt die Reinigung der Zahnhartsubstanz vorzugsweise unter Verwendung der beschriebenen Pulver bzw. Pulvergemische.

Schließlich kann der Erfolg der Reinigung erneut, beispielsweise über eine sogenannte diagnostische Abformmasse, überprüft werden.

War die Reinigung nicht erfolgreich, kann gegebenenfalls unmittelbar daran eine erneute Reinigung der Zahnhartsubstanz unter Verwendung der beschriebenen Pulver bzw. Pulvergemische erfolgen, ohne dass die Zahnhartsubstanz nennenswert geschädigt wird.

Im folgenden wird die Erfindung anhand von Beispielen weiter erläutert:

### Vorbereitung von Rinderzähnen und Durchführung der Messungen:

Pro Versuch wurden je drei frisch extrahierte Rinderzähne verwendet, deren Schmelzbereich nach Reinigung durch Abspülen mit entionisiertem Wasser oberflächlich durch Behandlung mit Schleifpapier geglättet wurde. Der so vorbereitete Rinderzahn wurde in einer Einbettmasse (Permagum®, Fa. ESPE, Seefeld) fixiert und mit einer Metallplatte abgedeckt, welche eine kreisrunde Aussparung mit einem Durchmesser von 3,5 mm aufwies. Die freiliegende Schmelzfläche wurde anschließend für zwei Minuten mittels eines Pulverstrahlgerätes (Airflow®, Fa. EMS, München) mit dem entsprechenden Pulver bzw. Pulvergemisch bei einem Strahldruck von 4,0 bar und einem Abstand zwischen Wurzeloberfläche zu Strahldüse von 2,3 mm bestrahlt. Für jeden Versuch wurden jeweils maximal befüllte Pulvertanks verwendet. Das Gerät wurde mit der Einstellung "Pulver voll" und "Wasser halb" betrieben.

Zur Ermittlung des abgetragenen Schmelzvolumens wurden die abgestrahlten Oberflächen mittels einer Abformmasse (Dimension® Garant, Fa. ESPE, Seefeld) dubliert. Das dabei in Form eines Halbelipsoids entstandene Negativ des abgetragenen Volumens wurde unter einem Lichtmikroskop (Zeiss Stereomikroskop, 40- bis 64-fache Vergrößerung) entlang seiner Achsen vermessen und anhand dieser Daten mit Hilfe folgender Formel der Volumenabtrag berechnet:
Abgetragenes Volumen = 2/3 π a * b * c

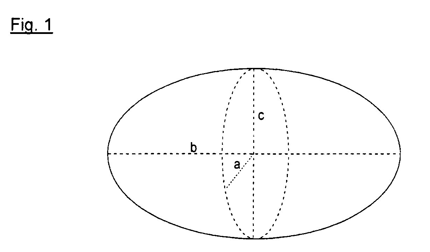

Der Halbelipsoid zur Berechnung des Volumenabtrags ist in Fig. 1 dargestellt.

### Beispiel I: Pulvermischung I

100 g Glycin (Fa. Fluka, Deisenhofen) wurden für 3 Minuten in einer Achatscheibenmühle gemahlen und anschließend trocken über ein 40 µm Sieb gesiebt. Anschließend wurde das so gewonnene Pulver mit 0,36 g HDK-H-2000 (Fa. Degussa, Hanau) versetzt und diese Mischung nochmals über ein 60 µm Sieb gesiebt.

### Beispiel II: Pulvermischung II

100 g Kalium-D-Glukonat (Fa. Fluka, Deisenhofen) wurden für 4 Minuten in einer Achatscheibenmühle gemahlen und anschließend trocken über ein 40 µm Sieb gesiebt. Anschließend wurde das so gewonnene Pulver mit 0,63 g HDK-H-2000 (Fa. Degussa, Hanau) versetzt und diese Mischung nochmals über ein 60 µm Sieb gesiebt.

### Beispiel III: Pulvermischung III

100 g Natriumascorbat (Fa. Fluka, Deisenhofen) wurden für 1 Minute in einer Achatscheibenmühle gemahlen und anschließend trocken über ein 40 µm Sieb gesiebt. Anschließend wurde das so gewonnene Pulver mit 0,9 g HDK-H-2000 (Fa. Degussa, Hanau) versetzt und diese Mischung nochmals über ein 60 µm Sieb gesiebt.

### Referenzbeispiel I:

100 g Natriumhydrogencarbonat (Fa. Fluka, Deisenhofen) wurden für 2,5 Minuten in einer Achatscheibenmühle gemahlen und anschließend trocken über ein 40 µm Sieb gesiebt. Anschließend wurde das so gewonnene Pulver mit 0,19 g HDK-H-2000 (Fa. Degussa, Hanau) versetzt und diese Mischung nochmals über ein 60 µm Sieb gesiebt.

### Referenzbeispiel II:

100g Air-Flow-Pulver (Fa. EMS) wurden wie vom Hersteller geliefert eingesetzt.

Die so gewonnenen Pulvermischungen 1-VI, sowie die Referenzpulver I und II wurden in ein Pulverstrahlgerät (Airflow®, Fa. EMS, München) gefüllt und wie vorher beschrieben verwendet. Die jeweilige Menge an abgetragener Zahnsubstanz ist der Tabelle 1 zu entnehmen.

**Tabelle 1: Abgetragenes Volumen an Rinderwurzeldentin in Abhängigkeit der verwendeten Pulvermischung bzw. deren Dichte und mittleren Korngröße.**

| Pulvermischung | Dichte [g/cm³]* | Mittlere Korngröße [µm]** | Abgetragenes Volumen [mm³] |
|---|---|---|---|
| I | 1.16 | 10,7 | nicht messbar |
| II | 1.73 | 21,7 | nicht messbar |
| III | 1.80 | 21,0 | 0,09 |
| Referenz I | 2.16 | 34,8 | 0,13 |
| Referenz II | 2,16 | 54,3 | 0,15 |

| | | | |
|---|---|---|---|
| * Quelle: Beilstein ** Gemessen an Granulometer der Fa. CILAS mit isopropanol als Dispergiermittel | | | |

Der in Tabelle 1 jeweils angegebene Volumenwert ergibt sich aus der Summe der ermittelten Volumen dividiert durch die Anzahl der vermessenen Zähne.

## Patentansprüche

1. Verwendung feinkörniger Pulver und/oder Pulvergemische zur Herstellung eines Mittels für die Pulverstrahlreinigung von supragingivalen Zahnoberflächen, wobei die durch Pulverbestrahlung verursachte Abrasion der supragingivalen Zahnoberfläche nicht mehr als 0,10 mm³ beträgt, bezogen auf eine Zahnoberfläche von 9,6 mm² bei einer Bestrahlungsdauer von 2 min bei einem Strahldruck von 4 bar und einem Abstand zwischen Zahnoberfläche und Strahldüse von nicht mehr als 2,5 mm, wobei die Pulver eine Dichte von nicht mehr als 2,0 g/cm³ und eine mittlere Korngröße von nicht mehr als 45 µm aufweisen und dem Pulver bzw. dem Pulvergemisch ein weiterer feinkörniger Stoff beigemischt ist.

2. Verwendung nach einem der vorstehenden Ansprüche, wobei die Pulver eine Kornverteilung im Bereich von 0,05 µm bis 60 µm aufweisen.

3. Verwendung nach einem der vorstehenden Ansprüche, wobei die Pulvergemische aus mindestens zwei Pulvern bestehen und das Mischungsverhältnis im Bereich von 1 : 10 bis 10 : 1 liegt, bezogen auf die Masse der zu mischenden Pulver.

4. Verwendung nach einem der vorstehenden Ansprüche, wobei der weitere feinkörnige Stoff in einer Menge von 0,01 bis 5,0 Gew.% zugesetzt ist.

5. Verwendung feinkörniger Pulver und/oder Pulvergemische nach Anspruch 5, wobei der feinkömige Stoff gewählt ist aus Boroxid, Kieselgel und/oder Kieselsäure, vorzugsweise silanisiert.

6. Verwendung nach einem der vorstehenden Ansprüche, wobei dem Pulver oder Pulvergemisch feinteilige Substanzen gewählt aus Bleichmitteln, fluoridfreisetzenden Substanzen, Analgetika, Bakteriozide oder Geschmacksstoffe zugesetzt sind.

7. Verwendung feinkörniger Pulver und/oder Pulvergemische nach einem der vorstehenden Ansprüche, wobei die Pulver bzw. Pulvergemische gewählt sind aus Aminosäuren, Zuckern, organischen Säuren und deren Salze.

8. Verwendung nach einem der vorstehenden Ansprüche, wobei die Pulver oberflächenbeschichtet sind.

9. Verwendung feinkörniger Pulver und/oder Pulvergemische nach einem der vorstehenden Ansprüche, wobei die Pulver bzw. Pulvergemische mittels eines Strahldruckgerätes aufgebracht werden.

10. Kit zur Reinigung von Zahnoberflächen, umfassend Pulver und/oder Pulvergemische, wie sie in den vorstehenden Ansprüchen beschrieben sind und gegebenenfalls ein System zur Detektion von Verunreinigungen und/oder Ablagerungen auf der Zahnhartsubstanz.

11. Kit gemäß Anspruch 10, wobei das System eine Abformmasse umfasst.

12. Strahldruckgerät enthaltend ein Pulver und/oder ein Pulvergemisch, wie es in den Ansprüchen 1 bis 9 beschrieben ist.

## Claims

1. Use of fine powders and/or powder mixtures for producing a composition for the powder jet cleaning of supragingival tooth surfaces, the abrasion to the supragingival tooth surface caused by powder jet cleaning being not more than 0.10 mm³, based on a tooth surface of 9.6 mm² for a jet cleaning duration of 2 min at a jet pressure of 4 bar, and a distance between tooth surface and jet nozzle of not more than 2.5 mm, wherein the powders have a density of not more than 2.0 g/cm³ and an average particle size of not more than 45 µm and a further finely particulate substance is admixed to the powder or powder mixture.

2. Use according to any of the above claims, wherein the powders have a particle distribution in the range from 0.05 to 60 µm.

3. Use according to any of the above claims, wherein the powder mixtures are composed of at least two powders and the mixing ratio is in the range from 1:10 to 10:6, based on the mass of the powders to be mixed.

4. Use according to any of the above claims, wherein the further finely particulate substance has been added in an amount of from 0.01 to 5.0% by weight.

5. Use of fine powders and/or powder mixtures according to Claim 5, wherein the finely particulate substance is selected from boron oxide, silica gel and/or silica, preferably silanized.

6. Use according to any of the above claims, wherein finely divided substances selected from bleaches, fluoride donor substances, analgesics, bacteriocides or flavours have been added to the powder or powder mixture.

7. Use of fine powders and/or powder mixtures according to any of the above claims, wherein the powders or powder mixtures are selected from amino acids, sugars, organic acids and salts thereof.

8. Use according to any of the above claims, wherein the powders have been surface-coated.

9. Use of fine powders and/or powder mixtures according to any of the above claims, wherein the powders and powder mixtures are applied by means of a jet pressure device.

10. Kit for cleaning tooth surfaces, comprising powders and/or powder mixtures, as described in the above claims, and, if desired, a system for detecting contaminations and/or deposits on the hard surface of the tooth.

11. Kit according to Claim 10, wherein the system comprises an impression compound.

12. Jet pressure device containing a powder and/or a powder mixture as described in Claims 1 to 5.

## Revendications

1. Utilisation de poudres et/ou de mélanges de poudres à grains fins pour la préparation d'un agent de nettoyage de la surface supragingivale des dents par projection de poudre, l'abrasion provoquée sur la surface supragingivale des dents par la projection de poudre n'étant pas supérieure à 0,10 mm³ pour une surface dentaire de 9,6 mm², une durée de projection de 2 min., une pression de projection de 4 bars et à une distance entre la surface de la dent et l'ajutage de projection non supérieure à 2,5 mm, la poudre présentant une masse spécifique non supérieure à 2,0 g/cm³, une granulométrie moyenne non supérieure à 45 µm, une autre substance en grains fins étant ajoutée à la poudre ou selon le cas au mélange de poudres.

2. Utilisation selon l'une des revendications précédentes, dans laquelle les poudres ont une distribution granulométrique comprise dans la plage de 0,05 µm à 60 µm.

3. Utilisation selon l'une des revendications précédentes, dans laquelle les mélanges de poudres sont constitués d'au moins deux poudres dans un rapport de mélange compris dans la plage de 1:10 à 10:1 par rapport à la masse des poudres mélangées.

4. Utilisation selon l'une des revendications précédentes, dans laquelle l'autre substance en grains fins est ajoutée en quantité de 0,01 à 5,0% en poids.

5. Utilisation de poudres et/ou de mélanges de poudres en grains fins selon la revendication 5, dans laquelle la substance en grains fins est sélectionnée parmi l'oxyde de bore, le gel de silice et/ou l'acide silicique, de préférence silanisé.

6. Utilisation selon l'une des revendications précédentes, dans laquelle on ajoute à la poudre ou au mélange de poudres des substances finement divisées sélectionnées parmi les agents de blanchissement, les substances qui libèrent des fluorures, les analgésiques, les bactéricides ou les substances organoleptiques.

7. Utilisation de poudres et/ou de mélanges de poudres en grains fins selon l'une des revendications précédentes, dans laquelle les poudres ou selon le cas mélanges de poudres sont sélectionnés parmi les acides aminés, les sucres, les acides organiques et leurs sels.

8. Utilisation selon l'une des revendications précédentes, dans laquelle les poudres sont revêtues en surface.

9. Utilisation de poudres et/ou de mélanges de poudres en grains fins selon l'une des revendications précédentes, dans laquelle les poudres ou selon le cas mélanges de poudres sont appliqués à l'aide d'un appareil de projection sous pression.

10. Trousse de nettoyage de la surface des dents, qui comprend des poudres et/ou des mélanges de poudres décrits dans les revendications précédentes et éventuellement un système de détection des impuretés et/ou des dépôts sur la substance dure des dents.

11. Trousse selon la revendication 10, dans laquelle le système comprend une pâte de moulage.

12. Appareil de projection sous pression qui contient une poudre et/ou un mélange de poudres décrits dans les revendications 1 à 5.
